# FASCICULE DE BREVET EUROPEEN

(11) **EP 2 055 334 B1**
(45) Date de publication et mention de la délivrance du brevet: **29.05.2013**
(21) Numéro de dépôt: 08166632.3
(22) Date de dépôt: 15.10.2008
(51) Int. Cl.: A61M 5/32, A61M 5/50

(54) **Dispositif de seringue à capuchon de protection**
Spritzenvorrichtung mit Schutzkappe
Syringe device with protective cap

(30) Priorité: 23.10.2007 FR 0758496
(43) Date de publication de la demande: 06.05.2009
(73) Titulaire: Tech Group Europe Limited, Dublin 2 (IE)
(72) Inventeur: Chevallier, Stéphane, 77165, SAINT-SOUPPLETS (FRANCE) (FR); Chevallier, Jean-Michel, 95880, ENGHIEN-LES-BAINS (FRANCE) (FR)
(74) Mandataire: Intès, Didier Gérard André

(56) Documents cités:
- EP-A- 1 532 997
- WO-A-03/077977
- WO-A-2006/027445
- FR-A- 2 762 790
- FR-A- 2 807 665

## Description

La présente invention concerne un dispositif de seringue comprenant un corps de seringue présentant une extrémité distale équipée d'une aiguille d'injection, au moins un fourreau à l'intérieur duquel ce corps est disposé, et un capuchon de protection, apte à occuper une position de protection dans laquelle le capuchon est retenu par rapport au corps de seringue de manière à coiffer l'aiguille et à être éloigné de ce corps pour dégager l'aiguille, le dispositif comportant un organe anti-retour, apte à s'opposer à une remise en place du capuchon dans sa position de protection une fois que l'aiguille a été dégagée.

Des dispositifs de ce type sont connus. Le fourreau peut être un simple fourreau de support, servant à maintenir en son intérieur le corps de seringue. Il peut également s'agir d'un dispositif de sécurité, auquel cas il peut comprendre un manchon de protection qui, en fin d'injection, est placé autour de l'aiguille par un coulissement relatif de ce manchon et du corps de seringue, de manière à éviter que l'utilisateur ne se pique avec cette aiguille. Dans ce cas, le fourreau précité peut être ce manchon de protection, ou bien un manchon de support servant à maintenir le corps de seringue, auquel cas le manchon de support et le manchon de protection sont aptes à coulisser l'un par rapport à l'autre.

Le corps de seringue utilisé dans le dispositif de seringue de l'invention est en particulier un corps de seringue pré-rempli, à usage unique. Dans ce cas, il convient de s'assurer avant de pratiquer une injection que la seringue n'a pas encore été utilisée. Il convient en particulier de s'assurer que l'aiguille n'a pas pu être souillée par une contamination extérieure, que ce soit par inadvertance ou à la suite d'une malveillance. Pour cela, il est nécessaire qu'un dispositif de seringue qui aurait été utilisé ou bien dont l'aiguille aurait été souillée ne se présente pas comme un dispositif de seringue "vierge" de toute utilisation précédente.

Pour faire en sorte que l'utilisateur du dispositif puisse aisément s'assurer que celui-ci n'a pas fait l'objet d'un précédent usage ou d'une souillure de l'aiguille, WO 2006/027445 prévoit d'équiper le fourreau protecteur de languettes qui, au repos, délimitent une ouverture inférieure à celle du capuchon, de manière à empêcher une remise en place aisée du capuchon. Il en va de même pour EP 1 532 997 et WO 03/077977.

FR 2 762 790 préconise l'utilisation de pétales formant des languettes qui, au repos, définissent un diamètre supérieur à celui d'un sillon dans lequel ces pétales doivent être engagés pour permettre la mise en place du capuchon.

Ce dispositif de l'art antérieur ne donne pas toute satisfaction car, selon le nombre de languettes, il est possible, manuellement, de toutes les contraindre à s'écarter, voire de casser l'une ou plusieurs d'entre elles pour remettre le capuchon en place.

L'invention a pour but d'améliorer cet état de la technique en choisissant la structure de l'organe anti-retour pour rendre la remise en place du capuchon quasiment impossible, ou du moins nettement plus difficile.

Ce but est atteint grâce au fait que l'organe anti-retour comprend une lèvre élastique qui est solidaire de l'un des éléments constitués par le fourreau et par le capuchon et qui forme un anneau fermé, ladite lèvre étant contrainte élastiquement lorsque le capuchon est dans sa position de protection et adoptant à l'état libre, lorsque le capuchon est éloigné du corps, une configuration apte à s'opposer à la remise en place du capuchon en position de protection.

Cette lèvre élastique peut être aisément rapportée sur l'élément qui la porte par surmoulage, collage, ou tout autre moyen de fixation permanente. Elle est donc très aisée à fabriquer et à mettre en place, tout en constituant un moyen simple et efficace de s'opposer à une remise en place du capuchon une fois que celui-ci a été ôté pour dégager l'aiguille.

La lèvre formant un anneau fermé, et donc continu, il faut, pour rendre son diamètre compatible avec la remise en place du capuchon, modifier ce diamètre uniformément. Ceci est quasiment impossible par une sollicitation manuelle puisque les doigts ne peuvent pas uniformément entourer une telle lèvre sur tout son pourtour pour uniformément modifier son diamètre.

Pour utiliser le dispositif ou souiller l'aiguille, il est nécessaire de séparer le capuchon du corps de seringue et de dégager l'aiguille. Une fois ceci effectué, la lèvre élastique de l'invention s'oppose efficacement à une remise en place du capuchon. L'utilisateur peut donc aisément vérifier avant une première utilisation, si le capuchon est correctement en place ou non, une non-remise en place signalant une utilisation préalable ou une souillure de l'aiguille.

Avantageusement, le capuchon coopère par emmanchement avec l'extrémité distale du fourreau et, à l'état libre, la lèvre élastique présente des dimensions diamétrales s'opposant à cet emmanchement.

Comme on le verra dans la suite, la lèvre peut être solidaire du capuchon, auquel cas ses dimensions diamétrales à l'état libre peuvent être trop grandes pour permettre son emmanchement dans le manchon. Elle peut à l'inverse être solidaire du fourreau, auquel cas ses dimensions diamétrales à l'état libre peuvent être trop petites pour permettre l'insertion du capuchon par emmanchement dans l'extrémité distale du fourreau.

Avantageusement, l'extrémité libre de la lèvre élastique est amincie.

Ceci améliore les propriétés de retour élastique de la lèvre en faisant en sorte que sa portion plus épaisse soit solidaire de l'élément qui la porte. La lèvre peut avoir en section sensiblement la forme d'une virgule.

Avantageusement, le fourreau est un manchon de support par rapport auquel le corps de seringue est retenu.

En variante, avantageusement, le fourreau est un manchon de protection, le corps de seringue et le manchon de protection étant aptes à coulisser l'un par rapport à l'autre, de manière à occuper une position d'attente, dans laquelle l'extrémité libre de l'aiguille dépasse par rapport au fourreau et une position de protection dans laquelle le fourreau est disposé autour de l'extrémité libre de l'aiguille.

Il est avantageux que le dispositif comporte un manchon de support par rapport auquel le corps de seringue est retenu, et un manchon de protection, le corps de seringue et le manchon de protection étant aptes à coulisser l'un par rapport à l'autre, de manière à occuper une position d'attente dans laquelle l'extrémité libre de l'aiguille dépasse par rapport au manchon de protection et une position de protection dans laquelle le manchon de protection est disposé autour de l'extrémité libre de l'aiguille.

Dans ce cas, le fourreau est avantageusement formé par l'un des éléments constitués par le manchon de support et par le manchon de protection.

L'invention sera bien comprise et ses avantages apparaîtront mieux à la lecture de la description détaillée qui suit, de modes de réalisation représentés à titre d'exemples non limitatifs. La description se réfère aux dessins annexés, sur lesquels :
- la figure 1 est une vue en coupe longitudinale d'un dispositif de seringue conforme à l'invention, avant sa première utilisation ;
- la figure 2 est une vue en coupe longitudinale, prise dans le plan II-II indiqué sur la figure 1 ;
- la figure 3 est un agrandissement de la zone III de la figure 1 ;
- la figure 4 montre la même zone que la figure 3, alors que le capuchon a été ôté et que l'on tente de le remettre en place ;
- les figures 5 et 6 sont des vues respectivement analogues aux figures 3 et 4 pour un autre mode de réalisation ; et
- les figures 7 et 8 sont des vues respectivement analogues aux figures 3 et 4, pour encore un autre mode de réalisation.

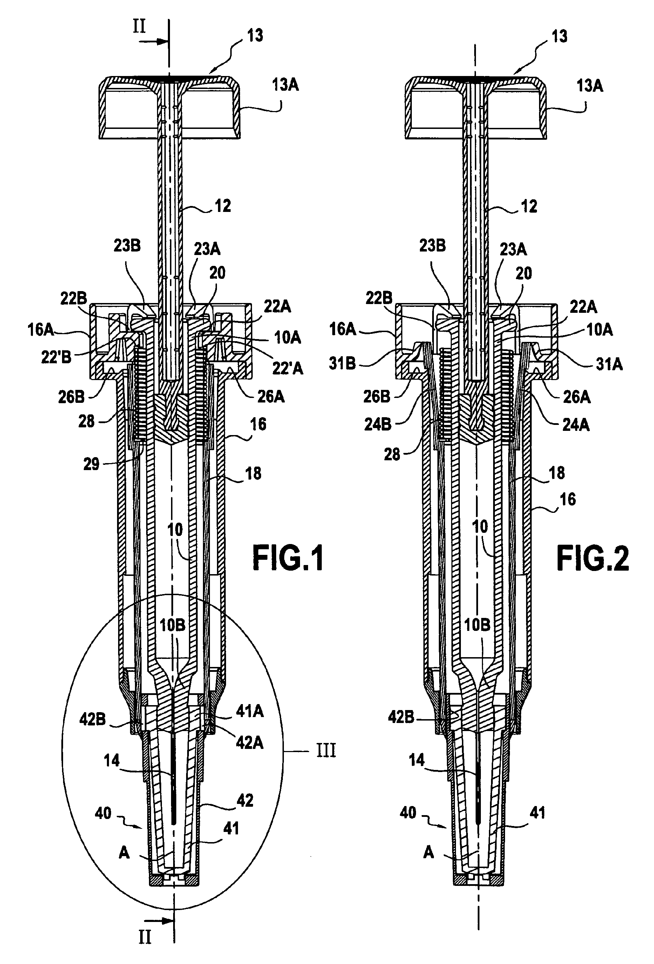

Le dispositif représenté sur les figures 1 et 2 comprend un corps de seringue 10 dans lequel un piston 12 peut coulisser en étant inséré dans ce corps à partir de son extrémité proximale 10A. A son extrémité distale 10B, le corps est équipé d'une aiguille d'injection 14. Le corps de seringue est par exemple en plastique ou en verre.

Au sens de la présente invention, l'extrémité proximale d'un élément est celle qui est la plus proche des doigts d'un utilisateur pratiquant une injection avec le dispositif, tandis que l'extrémité distale est l'extrémité opposée.

Dans l'exemple représenté, le dispositif de seringue est un dispositif de sécurité, du type décrit dans la demande de brevet PCT WO 03/068298. En effet, il comporte un manchon extérieur de support 16 par rapport auquel le corps de seringue 10 est retenu, et un manchon intérieur de protection 18 qui, en fin d'injection, peut coulisser entre le manchon extérieur de support et le corps de seringue pour venir se placer autour de l'aiguille 14 et protéger ainsi l'utilisateur contre des piqûres malencontreuses. Les figures montrent le manchon de protection en position escamotée à l'intérieur du manchon de support.

A son extrémité proximale 10A, le corps de seringue présente une collerette radiale 20 qui est calée entre, d'une part, deux portions d'épaulement 22A et 22B appartenant à une portion proximale 16A du manchon de support et, d'autre part, des pattes élastiques de retenue 23A et 23B appartenant également à cette partie 16A.

Par ailleurs, comme on le voit sur la figure 2, le manchon de protection 18 présente, à son extrémité proximale, des pattes de retenue 24A et 24B qui sont accrochées sur des surfaces d'épaulement 26A et 26B de la portion proximale 16A du manchon de support. Un ressort de rappel 28 est disposé entre un épaulement interne 29 du manchon de protection 18 et les faces internes 22'A et 22'B des épaulements 22A et 22B de la portion 16A du manchon 10. Par ailleurs, dans l'exemple représenté, des pattes d'actionnement 31A et 31B sont solidaires de la portion 16A. On comprend que, lorsque le piston 12 arrive en fin d'injection, la jupe 13A que présente sa tête 13 vient repousser les pattes d'actionnement 31A et 31B vers l'axe A du dispositif, repoussant également ainsi les pattes de retenue 24A et 24B vers cet axe, de manière à décrocher le manchon de protection 18 et permettre l'avancée de celui-ci vers sa position de protection sous l'effet de l'effort exercé par le ressort.

Le mécanisme qui vient d'être décrit pour assurer la sécurité du dispositif en venant entourer l'aiguille à la fin d'une injection ne l'a été qu'à titre d'exemple. De manière générale, on peut utiliser pour assurer la protection tout type de dispositif comprenant un manchon de support par rapport auquel le corps de seringue sera retenu et un manchon de protection, ces manchons étant agencés de telle sorte que, en fin d'injection, le manchon de protection et le corps de seringue puissent coulisser l'un par rapport à l'autre afin d'assurer la protection de l'aiguille. Par exemple, il est possible que l'ensemble constitué par le corps de seringue et par le manchon de support recule dans le sens allant de l'extrémité distale vers l'extrémité proximale à la fin d'une injection pour faire rentrer l'aiguille dans l'extrémité distale du manchon de protection. Le manchon de support peut être le seul fourreau et, en lui-même, servir à la protection, par exemple en permettant un recul du corps de seringue par rapport à ce fourreau en fin d'injection.

De manière générale, le dispositif de l'invention s'applique à tout type de dispositif de seringue dans lequel un corps de seringue est disposé à l'intérieur d'un fourreau, que ce fourreau serve ou non à assurer la protection. Comme on le verra dans la suite, le fourreau au sens de l'invention peut par exemple être constitué par le manchon de support ou bien par le manchon de protection qui vient d'être décrit, lorsque le dispositif comporte un agencement de protection.

Lorsque ce n'est pas le cas, le fourreau au sens de l'invention peut être constitué par un simple manchon de support.

Comme on le voit sur les figures 1 à 3, le dispositif de l'invention comprend un capuchon de protection 40 qui, sur ces figures, est représenté dans sa position de protection dans laquelle il est retenu par rapport au corps de seringue de manière à coiffer l'aiguille 14. En l'espèce, ce capuchon comprend une partie de capuchon interne 41 qui est emmanchée autour de l'extrémité distale 10B du corps de seringue et dont la seule ouverture est celle qui délimite son bord d'emmanchement 41A situé à son extrémité proximale. Classiquement, cette partie de capuchon interne 41 présente une relative élasticité, lui permettant d'être naturellement retenu par accrochage derrière un léger renflement 10'B de l'extrémité distale 10B du corps de seringue 10. Le capuchon présente encore une portion externe de capuchon 42 qui est solidaire de la portion interne 41. Plus précisément, la portion externe 42 présente des fenêtres latérales 42A, 42B, qui sont diamétralement opposées et dans lesquelles vient se bloquer un tronçon 41A de la portion 41, ce tronçon 41A présentant à cet effet un diamètre élargi. Entre les fenêtres 42A et 42B, la paroi 42C de la portion externe 42 est amincie.

Bien entendu, d'autres formes de capuchon sont envisageables. Le capuchon est un élément qui vient coiffer l'aiguille et présente donc, globalement, une forme de coiffe, adaptée à cette fonction. Il peut être en une seule partie ou en plusieurs parties. Il présente en outre une conformation permettant sa solidarisation amovible avec l'extrémité distale du corps de seringue.

Le dispositif de l'invention comporte un organe anti-retour qui s'oppose à une remise en place du capuchon 40 dans sa position de protection une fois que l'aiguille a été dégagée, c'est-à-dire une fois que ce capuchon a été séparé du corps de seringue pour permettre l'accès à l'aiguille, en particulier pour une injection.

Cet organe anti-retour comprend une lèvre élastique. Plus précisément, dans le mode de réalisation représenté sur les figures 1 à 4, une lèvre élastique 50 est solidaire d'une portion d'extrémité 18A du manchon de protection 18. On voit sur les figures 1 à 3 que, lorsque le capuchon 40 est dans sa position de protection, l'extrémité libre 50A de la lèvre est en appui contre la périphérie externe 40A du capuchon.

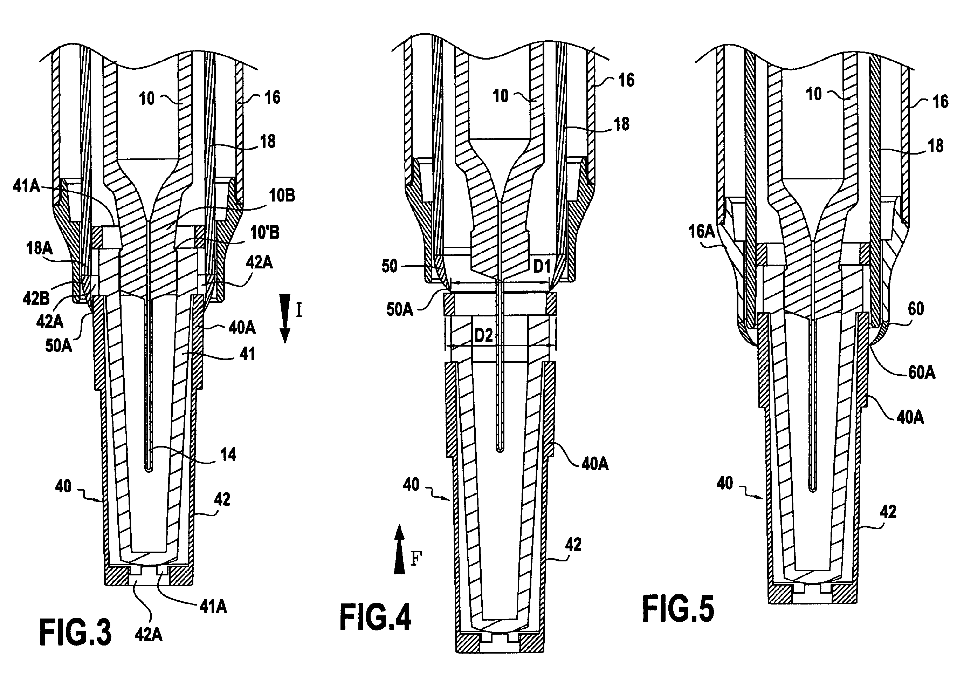

En revanche, sur la figure 4, le capuchon 40 a été dégagé, et l'extrémité libre 50A de la lèvre 50 a naturellement, sous l'effet de son élasticité, repris une configuration à l'état libre dans laquelle elle délimite des dimensions diamétrales D1 inférieures aux dimensions diamétrales D2 que présente la périphérie externe du capuchon dans sa zone d'engagement avec le fourreau (dans ce premier mode de réalisation, le fourreau est le manchon de protection 18). Ainsi, lorsque l'on tente de remettre en place le capuchon par emmanchement dans la portion d'extrémité distale du manchon de protection 18 par une poussée dans le sens F indiqué sur la figure 4, la lèvre 50 s'oppose à cette remise en place.

Il convient de relever que le corps de seringue est initialement pré-équipé du capuchon 40 et que ce corps de seringue ainsi que le capuchon présentent des dimensions diamétrales maximales permettant leur insertion dans l'ensemble constitué par le manchon de support et le manchon de protection par l'extrémité proximale de ces manchons, dans le sens de la flèche I indiquée sur la figure 3. Ainsi, la lèvre 50 ne s'oppose pas à cette mise en place, mais l'insertion du corps de seringue et du capuchon dans les manchons écarte élastiquement cette lèvre pour la contraindre élastiquement en expansion de telle sorte que son extrémité libre 50A vienne se plaquer contre la paroi externe du capuchon.

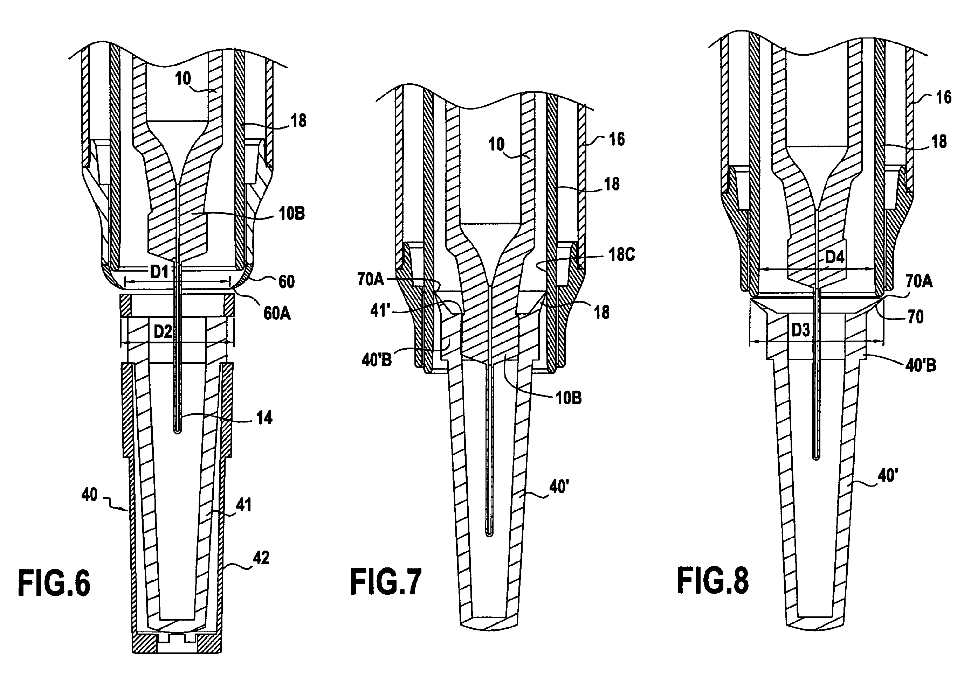

Sur les figures 5 et 6, la lèvre élastique 60 est solidaire de la portion d'extrémité distale 16A du manchon de support 16. Sur la figure 5, on voit que l'extrémité libre 60A de la lèvre est en appui contre la périphérie externe 40A du capuchon 40 lorsque le capuchon est dans sa position de protection, avant une première utilisation du dispositif. Cette lèvre est alors davantage incurvée que dans l'exemple des figures 1 à 4, pour combler le jeu correspondant à l'épaisseur du manchon intérieur de protection 18.

Sur la figure 6, le capuchon a été ôté pour que l'aiguille 14 soit dégagée, et on voit que l'extrémité libre 60A de la lèvre 60 délimite des dimensions diamétrales D1 inférieures aux dimensions diamétrales D2 que présente la périphérie externe du capuchon dans sa zone d'engagement avec le manchon 16, de telle sorte que cette lèvre s'oppose à la remise en place du capuchon.

Sur les figures 7 et 8, la lèvre élastique 70 est solidaire d'une portion d'extrémité proximale 40'B du capuchon 40'. En l'espèce, ce capuchon présente la forme d'une simple coiffe, et est réalisé en une seule partie. Sa forme est donc globalement analogue à celle de la portion interne 41 de capuchon 40 décrite dans les figures précédentes, à ceci près que son extrémité proximale porte la lèvre précitée. On voit, sur la figure 7, que le capuchon est retenu par rapport au corps de seringue en étant emmanché à force autour de l'extrémité distale 10B de ce corps, en étant retenu par un léger effet d'accrochage de son bourrelet interne 41' dans une gorge annulaire 10'B de ladite extrémité distale 10B. Dans cette position, la lèvre 70 est contrainte dans le sens d'une réduction de ses dimensions diamétrales. En effet, son extrémité libre 70A est en appui contre la périphérie interne 18C du manchon de protection 18.

Comme on le voit sur la figure 8, une fois que le capuchon 40' a été séparé du reste du dispositif, la lèvre 70 retrouve élastiquement sa configuration à l'état libre, dans laquelle son extrémité libre 70A délimite des dimensions diamétrales D3 supérieures aux dimensions diamétrales D4 que présente la périphérie interne 18C du manchon de protection 18 dans sa zone d'engagement avec cette lèvre. En conséquence, il n'est plus possible d'emmancher l'extrémité proximale du capuchon 40' à l'intérieur de ce manchon. Si on cherche à forcer la mise en place du capuchon, la lèvre 70 a tendance à se retourner sur elle-même et forme alors, sur le bord du capuchon, une surépaisseur qui s'oppose à l'emmanchement du capuchon au bout du dispositif.

Dans l'exemple qui vient d'être décrit, l'extrémité libre 70A de la lèvre 70 coopère avec la périphérie interne du manchon de protection 18 lorsque le capuchon est dans sa position de protection. Bien entendu, on pourrait choisir une configuration différente, en faisant en sorte que cette extrémité libre coopère plutôt avec la périphérie interne du manchon de support 16. Il suffirait pour cela que, dans sa position rétractée à l'intérieur du manchon de support, le manchon de protection soit moins avancé vers l'extrémité distale.

De manière générale, le fourreau qui porte la lèvre élastique (modes de réalisation des figures 1 à 6) ou qui coopère avec la lèvre élastique portée par le capuchon (mode de réalisation des figures 7 et 8) peut être le manchon de support ou bien le manchon de protection.

La lèvre élastique est avantageusement réalisée en matériau plastique lui conférant l'élasticité requise. Il s'agit par exemple d'un matériau du type élastomère ou caoutchouc.

Dans le cas des figures 7 et 8, la lèvre 70 peut être réalisée en une seule pièce avec le reste du capuchon 40', par un moulage monobloc (auquel cas l'ensemble du capuchon est réalisé dans le même matériau), ou bien rapportée sur ce capuchon, par exemple par surmoulage ou collage.

## Revendications

1. Dispositif de seringue comprenant un corps de seringue (10) présentant une extrémité distale (10B) équipée d'une aiguille d'injection (14), au moins un fourreau (16, 18) à l'intérieur duquel ce corps est disposé, et un capuchon de protection (40, 40'), apte à occuper une position de protection dans laquelle le capuchon est retenu par rapport au corps de seringue (10) de manière à coiffer l'aiguille et à être éloigné de ce corps pour dégager l'aiguille, le dispositif comportant un organe anti-retour (50, 60, 70), contraint élastiquement lorsque le capuchon est dans sa position de protection et apte à adopter à l'état libre, lorsque le capuchon est éloigné du corps, une configuration dans laquelle ledit organe anti-retour est apte à s'opposer à une remise en place du capuchon (40, 40') dans sa position de protection une fois que l'aiguille a été dégagée,
**caractérisé en ce que** l'organe anti-retour comprend une lèvre élastique (50, 60, 70) qui est solidaire de l'un des éléments constitués par le fourreau (16, 18) et par le capuchon (40, 40') et qui forme un anneau fermé, ladite lèvre étant contrainte élastiquement lorsque le capuchon est dans sa position de protection et adoptant à l'état libre, lorsque le capuchon est éloigné du corps, une configuration apte à s'opposer à la remise en place du capuchon en position de protection.

2. Dispositif selon la revendication 1, **caractérisé en ce que**, en position de protection, le capuchon (40, 40') coopère par emmanchement avec l'extrémité distale (16A, 18A) du fourreau (16, 18) et **en ce que**, à l'état libre, la lèvre élastique (50, 60, 70) présente des dimensions diamétrales (D1, D3) s'opposant à cet emmanchement.

3. Dispositif selon la revendication 2, **caractérisé en ce que** la lèvre élastique (50, 60) est solidaire d'une portion d'extrémité distale (18A, 16A) du fourreau (18, 16) et présente une extrémité libre (50A, 60A) qui, lorsque le capuchon (40) est dans sa position de protection, est en appui contre la périphérie externe (40A) du capuchon (40), tandis que, à l'état libre de la lèvre élastique (50, 60), ladite extrémité libre (50A, 60A) délimite des dimensions diamétrales (D1) inférieures aux dimensions diamétrales (D2) de la périphérie externe (40A) du capuchon (40).

4. Dispositif selon la revendication 2, **caractérisé en ce que** la lèvre élastique (70) est solidaire d'une portion d'extrémité proximale (40'B) du capuchon (40') et présente une extrémité libre (70A) qui, lorsque le capuchon est dans sa position de protection, est en appui contre la périphérie interne (18C) du fourreau (18), tandis que, à l'état libre de la lèvre élastique (70), ladite extrémité libre (70A) délimite des dimensions diamétrales (D3) supérieures aux dimensions diamétrales (D4) de la périphérie interne (18C) du fourreau (18).

5. Dispositif selon la revendication 3 ou 4, **caractérisé en ce que** l'extrémité libre (50A, 60A, 70A) de la lèvre élastique (50, 60, 70) est amincie.

6. Dispositif selon l'une quelconque des revendications 1 à 5, **caractérisé en ce que** le fourreau est un manchon de support (16) par rapport auquel le corps de seringue (10) est retenu.

7. Dispositif selon l'une quelconque des revendications 1 à 5, **caractérisé en ce que** le fourreau est un manchon de protection (18), le corps de seringue (10) et le manchon de protection (18) étant aptes à coulisser l'un par rapport à l'autre, de manière à occuper une position d'attente, dans laquelle l'extrémité libre de l'aiguille (14) dépasse par rapport au fourreau et une position de protection dans laquelle le fourreau est disposé autour de l'extrémité libre de l'aiguille (14).

8. Dispositif selon l'une quelconque des revendications 1 à 7, **caractérisé en ce qu'**il comporte un manchon de support (16) par rapport auquel le corps de seringue (10) est retenu, et un manchon de protection (18), le corps de seringue (10) et le manchon de protection (18) étant aptes à coulisser l'un par rapport à l'autre, de manière à occuper une position d'attente dans laquelle l'extrémité libre de l'aiguille (14) dépasse par rapport au manchon de protection (18) et une position de protection dans laquelle ledit manchon est disposé autour de l'extrémité libre de l'aiguille (14).

9. Dispositif selon la revendication 8, **caractérisé en ce que** le fourreau est formé par l'un des éléments constitués par le manchon de support (16) et par le manchon de protection (18).

## Patentansprüche

1. Spritzenvorrichtung, umfassend einen Spritzenkörper (10), der ein mit einer Injektionsnadel (14) versehenes distales Ende (10B) aufweist, wenigstens eine Hülse (16, 18), in der dieser Körper angeordnet ist, sowie eine Schutzkappe (40, 40'), die geeignet ist, eine Schutzstellung einzunehmen, in der die Kappe gegenüber dem Spritzenkörper (10) fest gehalten ist, um die Nadel zu bedecken, und von diesem Körper entfernt zu werden, um die Nadel aufzudecken, wobei die Vorrichtung ein Rückkehrsicherungsorgan (50, 60, 70) umfaßt, das elastisch gespannt ist, wenn die Kappe sich in ihrer Schutzstellung befindet, und das geeignet ist, im freien Zustand, wenn die Kappe von dem Körper entfernt ist, eine Ausführung anzunehmen, in der das Rückkehrsicherungsorgan geeignet ist, sich einem Wiederanbringen der Kappe (40, 40') in ihre Schutzstellung entgegenzusetzen, sobald die Nadel einmal aufgedeckt worden ist,
**dadurch gekennzeichnet, daß** das Rückkehrsicherungsorgan eine elastische Lippe (50, 60, 70) umfaßt, die mit einem der durch die Hülse (16, 18) und durch die Kappe (40, 40') gebildeten Elemente fest verbunden ist und die einen geschlossenen Ring bildet, wobei die Lippe elastisch gespannt ist, wenn die Kappe sich in ihrer Schutzstellung befindet, und im freien Zustand, wenn die Kappe von dem Körper entfernt ist, eine Ausführung annimmt, die geeignet ist, sich dem Wiederanbringen der Kappe in Schutzstellung entgegenzusetzen.

2. Vorrichtung nach Anspruch 1, **dadurch gekennzeichnet, daß** die Kappe (40, 40') in Schutzstellung mit dem distalen Ende (16A, 18A) der Hülse (16, 18) durch Aufstecken zusammenwirkt und daß im freien Zustand die elastische Lippe (50, 60, 70) Durchmesserabmessungen (D1, D3) aufweist, die diesem Aufstecken entgegenwirken.

3. Vorrichtung nach Anspruch 2, **dadurch gekennzeichnet, daß** die elastische Lippe (50, 60) mit einem Abschnitt des distalen Endes (18A, 16A) der Hülse (18, 16) fest verbunden ist und ein freies Ende (50A, 60A) aufweist, das, wenn sich die Kappe (40) in ihrer Schutzstellung befindet, an dem Außenumfang (40A) der Kappe (40) in Anlage ist, während im freien Zustand der elastischen Lippe (50, 60) das freie Ende (50A, 60A) Durchmesserabmessungen (D1) begrenzt, die kleiner als die Durchmesserabmessungen (D2) des Außenumfangs (40A) der Kappe (40) sind.

4. Vorrichtung nach Anspruch 2, **dadurch gekennzeichnet, daß** die elastische Lippe (70) mit einem Abschnitt des proximalen Endes (40'B) der Kappe (40') fest verbunden ist und ein freies Ende (70A) aufweist, das, wenn die Kappe sich in ihrer Schutzstellung befindet, an dem Innenumfang (18C) der Hülse (18) in Anlage ist, während im freien Zustand der elastischen Lippe (70) das freie Ende (70A) Durchmesserabmessungen (D3) begrenzt, die größer als die Durchmesserabmessungen (D4) des Innenumfangs (18C) der Hülse (18) sind.

5. Vorrichtung nach Anspruch 3 oder 4, **dadurch gekennzeichnet, daß** das freie Ende (50A, 60A, 70A) der elastischen Lippe (50, 60, 70) verjüngt ist.

6. Vorrichtung nach einem der Ansprüche 1 bis 5, **dadurch gekennzeichnet, daß** die Hülse eine Haltemanschette (16) ist, der gegenüber der Spritzenkörper (10) fest gehalten ist.

7. Vorrichtung nach einem der Ansprüche 1 bis 5, **dadurch gekennzeichnet, daß** die Hülse eine Schutzmanschette (18) ist, wobei der Spritzenkörper (10) und die Schutzmanschette (18) geeignet sind, relativ zueinander zu gleiten, um eine Wartestellung, in der das freie Ende der Nadel (14) gegenüber der Hülse vorsteht, sowie eine Schutzstellung einzunehmen, in der die Hülse um das freie Ende der Nadel (14) herum angeordnet ist.

8. Vorrichtung nach einem der Ansprüche 1 bis 7, **dadurch gekennzeichnet, daß** sie eine Haltemanschette (16), der gegenüber der Spritzenkörper (10) fest gehalten ist, sowie eine Schutzmanschette (18) umfaßt, wobei der Spritzenkörper (10) und die Schutzmanschette (18) geeignet sind, relativ zueinander zu gleiten, um eine Wartestellung, in der das freie Ende der Nadel (14) gegenüber der Schutzmanschette (18) vorsteht, sowie eine Schutzstellung einzunehmen, in der die Manschette um das freie Ende der Nadel (14) herum angeordnet ist.

9. Vorrichtung nach Anspruch 8, **dadurch gekennzeichnet, daß** die Hülse von einem der durch die Haltemanschette (16) und durch die Schutzmanschette (18) gebildeten Elemente gebildet ist.

## Claims

1. A syringe device comprising a syringe body (10) having a distal end (10B) fitted with an injection needle (14), at least one sheath (16, 18) inside which the body is positioned, and a protective cap (40, 40'), able to occupy a protection position in which the cap is retained relative to the syringe body (10) so as to cover the needle and be separate from this body to release the needle, the device comprising a non-return member (50, 60, 70), which is elastically constrained when the cap is in its protection position and which is able to adopt, in the free state, when the cap is separated from the body, a configuration in which said non-return member is able to oppose a refitting of the cap (40, 40') in its protection position once the needle has been released,
**characterized in that** the non-return member comprises an elastic lip (50, 60, 70) which is joined to one of the elements formed by the sheath (16, 18) and by the cap (40, 40') and which forms a closed ring, said lip being elastically constrained when the cap is in its protection position and adopting, in the free state, when the cap is separated from the body, a configuration able to oppose the refitting of the cap in the protection position.

2. The device as claimed in claim 1, **characterized in that**, in the protection position, the cap (40, 40') cooperates by press-fitting with the distal end (16A, 18A) of the sheath (16, 18) and, in the free state, the elastic lip (50, 60, 70) has diametral dimensions (D1, D3) opposing this press-fitting.

3. The device as claimed in claim 2, **characterized in that** the elastic lip (50, 60) is joined to a distal end portion (18A, 16A) of the sheath (18, 16) and has a free end (50A, 60A) which, when the cap (40) is in its protection position, bears against the external periphery (40A) of the cap (40), whereas, in the free state of the elastic lip (50, 60), said free end (50A, 60A) delimits diametral dimensions (D1) less than the diametral dimensions (D2) of the external periphery (40A) of the cap (40).

4. The device as claimed in claim 2, **characterized in that** the elastic lip (70) is joined to a proximal end portion (40'B) of the cap (40') and has a free end (70A) which, when the cap is in its protection position, bears against the internal periphery (18C) of the sheath (18), whereas, in the free state of the elastic lip (70), said free end (70A) delimits diametral dimensions (D3) greater than the diametral dimensions (D4) of the internal periphery (18C) of the sheath (18).

5. The device as claimed in claim 3 or 4, **characterized in that** the free end (50A, 60A, 70A) of the elastic lip (50, 60, 70) is thinned.

6. The device as claimed in any one of claims 1 to 5, **characterized in that** the sheath is a support sleeve (16) relative to which the syringe body (10) is retained.

7. The device as claimed in any one of claims 1 to 5, **characterized in that** the sheath is a protection sleeve (18), the syringe body (10) and the protection sleeve (18) being able to slide relative to each other, so as to occupy a waiting position, in which the free end of the needle (14) extends beyond the sheath and a protection position in which the sheath is positioned around the free end of the needle (14).

8. The device as claimed in any one of claims 1 to 7, **characterized in that** it comprises a support sleeve (16) relative to which the syringe body (10) is retained, and a protection sleeve (18), the syringe body (10) and the protection sleeve (18) being able to slide relative to each other, so as to occupy a waiting position in which the free end of the needle (14) extends beyond the protection sleeve (18) and a protection position in which said sleeve is positioned around the free end of the needle (14).

9. The device as claimed in claim 8, **characterized in that** the sheath is formed by one of the elements comprising the support sleeve (16) and the protection sleeve (18).
